(19) Europäisches Patentamt
European Patent Office
Office européen des brevets

(11) **EP 1 794 550 B1**

(12) **EUROPÄISCHE PATENTSCHRIFT**

(45) Veröffentlichungstag und Bekanntmachung des Hinweises auf die Patenterteilung:
**20.09.2017 Patentblatt 2017/38**

(51) Int Cl.:
***C12M 1/34*** *(2006.01)*        ***G01F 3/38*** *(2006.01)*

(21) Anmeldenummer: **05791391.5**

(22) Anmeldetag: **28.09.2005**

(86) Internationale Anmeldenummer:
**PCT/DE2005/001725**

(87) Internationale Veröffentlichungsnummer:
**WO 2006/034699 (06.04.2006 Gazette 2006/14)**

(54) **BIOGAS-MESSEINRICHTUNG UND VERFAHREN ZUR MESSUNG VON BIOGASVOLUMEN**

BIOGAS MEASURING DEVICE AND METHOD FOR MEASURING BIOGAS VOLUMES

DISPOSITIF DE MESURE DE BIOGAZ ET PROCEDE DE MESURE DE VOLUME DE BIOGAZ

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.09.2004 DE 102004047560**

(43) Veröffentlichungstag der Anmeldung:
**13.06.2007 Patentblatt 2007/24**

(73) Patentinhaber: **Common-Link AG**
**76189 Karlsruhe (DE)**

(72) Erfinder: **SCHLÄFER, Ottmar**
**38709 Wildemann (DE)**

(74) Vertreter: **Gerstein, Hans Joachim**
**Gramm, Lins & Partner**
**Patent- und Rechtsanwälte PartGmbB**
**Theodor-Hauss-Strasse 1**
**38122 Braunschweig (DE)**

(56) Entgegenhaltungen:
**EP-A- 0 021 470        EP-A- 0 497 414**
**DE-A1- 3 540 481        DE-A1- 10 162 286**

**Beschreibung**

Die Erfindung betrifft eine Biogas-Messeinrichtung mit

- einem Bioreaktor,
- einem kommunizierend mit dem Bioreaktor über eine Gasleitung für Biogas verbundenen Gasvolumen-Messgerät mit:

  - einer Messkammer, die ein definiertes Messvolumen in einem Gehäuse hat,
  - einem mit einem Gaseinlassventil gesteuerten Gaseinlass in die Messkammer,
  - einem mit einem Gasauslassventil gesteuerten Gasauslass aus der Messkammer,
  - einem Drucksensor zur Erfassung des atmosphärischen Drucks,
  - einem mit der Messkammer kommunizierenden Differenzdrucksensor zur Erfassung des Differenzdrucks zwischen dem Gasdruck in der Messkammer und dem atmosphärischen Druck oder einem aktuellen Systemdruck,

und mit

- einer Mess-Steuer- und Auswerteeinheit, die zur Steuerung der Biogas-Messeinrichtung und Ermittlung der im Bioreaktor erzeugten Gasvolumen in Abhängigkeit von mit dem Gasvolumen-Messgerät gemessenen Gasvolumen aufeinander folgender Messzyklen mit den Schritten für jeden Messzyklus von:

    a) Einleiten einer Gasmenge in die Messkammer durch Öffnen des Gaseinlassventils solange, bis ein definierter Schwellen-Gasdruck erreicht ist,
    b) Schließen des Gaseinlassventils, wenn ein definierter Schwellen-Gasdruck erreicht ist,
    c) Messen der Temperatur in der Messkammer,
    d) Öffnen des Gasauslassventils für eine Entspannungszeit, und
    e) Schließen des Gasauslassventils

    eingerichtet ist.

Die Erfindung betrifft weiterhin ein Verfahren zur Messung von in einem Bioreaktor erzeugten Biogasvolumen mit einer Vielzahl von Messzyklen mit einer solchen Biogas-Messeinrichtung.

Gasvolumen-Messgeräte sind in Form von Verdrängungsgaszählern, Wirkdruckgaszählern und Strömungsgaszählern bekannt.

Verdrängungsgaszähler sind volumetrische Messgeräte, bei denen die Volumenmessung unmittelbar durch periodisches Füllen und Entleeren einer oder mehrerer Messkammern erfolgt. Hierbei wird die physikalische Eigenschaft des Gases genutzt, jeden umgrenzenden Raum vollständig auszufüllen. Die Teilvolumina oder Füllungen der Messkammern werden von einem Zählwerk addiert. Das hindurchgeströmte Gasvolumen entspricht dabei der Zahl der Füllungen mal dem Messkammerinhalt.

Alle Verdrängungsgaszähler sind unabhängig vom Strömungsprofil. Sie messen das Gasvolumen im Betriebszustand, also mit den tatsächlichen Werten für Druck und Temperatur. Als Antriebskraft für diese Gaszähler dient die Druckdifferenz zwischen Ein- und Ausgang des Messgerätes. Die Antriebsenergie wird dem Gasstrom entnommen. In diese Kategorie fallen die nassen Trommelgaszähler, die wegen ihrer hohen Präzision und des kleinen Messbereiches vorwiegend im Labor- und Kalibrierbereich eingesetzt werden. Für den industriellen Einsatz sind Trommelgaszähler zu teuer und auf Grund der täglich notwendigen Kontrolle der Sperrflüssigkeit zu wartungsintensiv. Eine Alternative stellt der Balgengaszähler dar. Als Verdrängungsgaszähler sind weiterhin Drehkolben- und Drehschleusengaszähler bekannt, die sehr robust sind, jedoch durch die bauartbedingten Spaltverluste und die erzeugten Pulsationen nur für hohe Gasvolumenströme geeignet sind.

Wirkdruckgaszähler bestehen aus einer Messstrecke mit Wirkdruckgeber (Blende oder Düse) und einem Durchfluss-Messgrößenumformer. An den in die Rohrleitung eingebauten Drosselgeräten ergibt sich in Folge der Querschnittsverengung eine Erhöhung der Strömungsgeschwindigkeit. Die hierdurch entstehende Druckdifferenz (Wirkdruck) ist ein Maß für den Durchfluss.

Bei Strömungsgaszählern erfolgt die Volumenmessung mittelbar durch von des Strömung bewegte oder periodisch beeinflusste, unbewegliche Messorgane. Die Volumenmessung wird auf eine Geschwindigkeitsmessung zurückgeführt. Es wird zwischen Turbinenradzählern, bei denen ein mit Schaufeln versehenes Rad durch Strömungskräfte in Drehung versetzt wird, und Wirbelgaszählern unterschieden, bei denen durch entsprechende Einbauten erzeugte periodische Strömungswirbel für die Zählung benutzt werden. Die Drehfrequenz des Turbinenrades beziehungsweise die Wirbelfrequenzen sind proportional zur mittleren Strömungsgeschwindigkeit und damit zum Volumendurchfluss. Zu den Strömungsgaszählern werden auch Ultraschallzähler gerechnet.

Bei der Vergärung von Biomasse erfolgt die Gasentwicklung entsprechend dem biologischen Abbau der organischen Verbindungen und der Zunahme der gasproduzierenden Zellmasse in einem "S"-förmigen Kurvenverlauf. Nach einem steilen Anstieg der Gasentwicklung bis zu einem Maximum folgt ein Abflachen der Gaskurve durch den Rückgang der Gasentwicklung zum Ende der Vergärung. Daraus entwickelt sich ein Volumenstrom, der im Laufe der Vergärung um mehrere Zehner Potenzen variiert und zum Ende der Vergärung nur noch wenige Milliliter pro Tag (ml/d) beträgt. Ein solch geringer Volumenstrom ist wegen der Verluste durch Spaltmaße, innere Widerstände und unbeständiger Strömung nicht durch offene Systeme wie Wirkdruck- und Strömungszähler, Drehschleusen- und Drehkolbenzähler zu erfassen. Auch die bekannten geschlossenen Trommel- und Balgenmessgeräte sind auf Grund des weiten Messbereiches und der benötigten Antriebsenergie der Systeme aus dem Gasstrom und der damit verbundenen Druckerhöhung in einem Versuchsreaktor ungeeignet.

Eine Besonderheit bei der Gasmessung kleiner maximaler Volumenströme von etwa $q_{max}<650$ m³/h und bei kleinen Effektivdrücken von etwa $P_{eff}<100$ hPa ist der Umstand, dass die Ermittlung des Normvolumes $V_n$ nicht durch einen Umwerter erfolgen muss, sonder allein durch Umrechnungen des Betriebsvolumens mit Korrekturfaktoren erreicht werden kann.

In Büchs J. und Anderlei, T.: "RAMOS (Respiration Activity Monitoring System) - Online - Messung der Atmungsaktivitäten biologischer Kulturen in geschüttelten Bioreaktoren, in BIOforum 3/2001, Seiten 149 bis 151 ist ein Verfahren zur Erfassung der Atmungsaktivität biologischer Kulturen in geschüttelten Bioreaktoren beschrieben. Während eines Kulturversuches in den Schüttelkolben wird kontinuierlich ein sich wiederholender Messzyklus durchlaufen, der in eine Mess- und eine Spülphase unterteilt ist. In der Spülphase sind ein Ein- und Auslassventil geöffnet und der Messkolben wird mit Spülgas durchströmt. Zur Beginn der Messphase werden das Ein- und Auslassventils des Messkolbens geschlossen. Die anhaltende Atmungsaktivität der Mikroorganismen führt zu Veränderungen des Sauerstoff- und Kohlendioxid-Partialdrucks im Gasraum des Messkolbens. Aus den Partialdruckänderungen, die mit Hilfe eines Sauerstoff- und Kohlendioxidsensors erfasst werden, wird eine Sauerstoff- (OTR) und Kohlendioxidtransferrate (CTR) sowie ein Respirationssquotient (RQ) im Messkolben bestimmt. Anschließend werden die Ventile wieder geöffnet und der nächste Messzyklus beginnt. Ein Gasvolumen, das von der Biomasse erzeugt würde, kann nicht ermittelt wurden.

Bei der Gasvolumenmessung insbesondere zur Optimierung der Biogasausbeute bei der Vergärung von Biomasse in einem Bioreaktor ist ein relativ großer Messbereich auf Grund der Charakteristik der biologischen Gasfreisetzung in diskontinuierlichen Versuchen erforderlich, um zu Beginn der Vergärung größere Volumenströme im Milliliter-Bereich pro Minute messen zu können und zum Ende geringe Volumenänderungen von wenigen Millilitern pro Tag. Dabei darf der biologische Prozess nicht beeinflusst werden. Insbesondere darf kein nennenswerter Druck im Versuchsreaktor entstehen. Auf Grund des daraus resultierenden geringen Druckgefälles, des geringen Volumenstroms und der hohen Anforderungen an die Langzeitdichtigkeit sind sämtliche mechanisch vom Gasstrom angetriebene offene Gasmesssysteme ungeeignet.

In dem US-Patent 5,742,523 A ist eine Messeinrichtung für Gasvolumen mit einem Gasbehälter mit bekanntem Volumen offenbart, der ein Einlassventil für Gas hat, um den Kessel mit Gas bis zum Erreichen eines Anfangsdrucks aufzufüllen. Über ein Ausgangsventil und eine Gasleitung wird nach Messung der Temperatur des Behälters und des Gasdrucks in dem Behälter der Gasdruck in dem Behälter berechnet und das Auslassventil bis zum Erreichen des berechneten Gasdrucks geöffnet. Dabei wird die Gastemperatur konstant gehalten. Auf diese Weise kann eine ganz genau festgelegte Masse von Gas durch das Auslassventil abgegeben werden.

Aus der FR 2 767 206 ist eine Gasmesseinrichtung bekannt, bei der über ein Einlassventil eine Gasmenge in ein definiertes Volumen gebracht wird. Durch Messung der Temperatur und des Gasdrucks in dem Volumen wird die Gasmenge in dem Volumen bestimmt.

Aus der DE 101 62 286 A1 ist weiterhin eine Vorrichtung zur Bestimmung des Gasvolumens bei Umgebungsdruck mit einer Messkammer, einem Drucksensor zur Erfassung des Innendrucks in der Messkammer, einem Drucksensor zur Erfassung des Umgebungsdruckes, und einem Temperatursensor beschrieben. Mit Hilfe von Ein- und Auslassventilen werden Gasvolumina durch die Messkammer geschleust und deren jeweilige Gasvolumina unter Anwendung des Gesetzes von Boyle nach der Formel $p_1 *V_1 / T_1 = p_2 *V_2 / T_2$ bestimmt, wobei $p_1$ der in der Messkammer herrschende Druck, $V_1$ das Volumen der Messkammer, $T_1$ die Temperatur in der Messkammer, $p_2$ der Umgebungsdruck, $V_2$ das gesuchte Gasvolumen und $T_2$ die Umgebungstemperatur ist.

EP 0 021 470 A1 offenbart einen Hohlrührer zum Eintreiben von Gas in eine Flüssigkeit mit einem aus 90°-Rohrbögen bestehendem Rotor.

EP 497 414 A beschreibt ein Verfahren zur möglichst schnellen Bestimmung des Abbaus eines organischen Produkts unter anaeroben Bedingungen. Das produzierte Biogas wird durch eine Rohrleitung in einen Gasprobenkolben geleitet, von wo es in einen Kondensationskolben geführt wird, um Kondensfeuchtigkeit aus dem gekühlten Biogas zu sammeln. Das Biogas wird dann weiter in einer Fühleinheit gekühlt und als derart getrocknetes Biogas über eine weitere Rohrleitung in ein Gasmessgerät geführt, indem das Volumen des erzeugten Biogases festgestellt wird.

Aufgabe der Erfindung ist es, eine Biogas-Messeinrichtung zu schaffen, mit dem in einem Biogasreaktor über einen langen Beobachtungszeitraum erzeugte Biogasvolumen hochgenau ermittelt werden können.

Die Aufgabe wird durch die Biogas-Messeinrichtung mit den Merkmalen des Anspruchs 1 gelöst.

Mit einer solchen Biogas-Messeinrichtung können aus einem hermetisch abgeschlossenen System einzelne Volumenelemente zuerst gemessen und anschließend entnommen werden, wobei die Druckverhältnisse annähernd konstant gehalten werden können. Dabei wird ein aus der DE 101 62 286 A1 bekanntes Gasvolumen-Messgerät mit einem Messprinzip genutzt, das im Wesentlichen auf einer Messkammer beruht, die über Gasleitungen direkt mit einem Bioreaktor verbunden werden kann und wobei durch einen empfindlichen

Drucksensor der Innendruck in der Messkammer gemessen wird. Wenn der Druck im System einen eingestellten, geringen Schwellenwert erreicht, wird die Messkammer durch ein Ventil vom Probenraum, dass heißt vom Bioreaktor getrennt und das eingeschlossene Gasvolumen wird bestimmt. Anschließend wird die Zelle durch Öffnen eines Gasauslassventils auf Umgebungsdruck entspannt und das Gas aus dem System freigesetzt. Nach dem Schließen des Auslassventils wird die Verbindung zum Bioreaktor wieder hergestellt und der Messzyklus kann von vorne beginnen.

Die erfindungsgemäße Biogas-Messeinrichtung ist ein abgeschlossenes Messsystem zur Biogaserzeugung, vorzugsweise im Labormaßstab, bei dem das Gasdruck-Messgerät mit einem Bioreaktor kommunizierend verbunden ist und das System nach außen gasdicht so abgeschlossen ist, dass sämtliche in dem Bioreaktor erzeugten Gasvolumina durch die Messkammer des Gasvolumen-Messgeräts erfassbar sind und kontrolliert durch das Gasvolumen-Messgerät ausgeschleust werden. Ein Rührwerk ist hierbei gasdicht in dem Bioreaktor geführt, um die Biomasse im Bioreaktor zu mischen. Weiterhin ist ein Kühlelement an der vom Bioreaktor zum Gasvolumen-Messgerät geführten Gasleitung für Biogas vorgesehen, um die Wasserdampfkonzentration im Biogas durch Kondensation zu verringern und hierdurch vergleichbare Messbedingungen zu gewährleisten.

Weiterhin wird durch die Normierung auf Standardbedingungen erreicht, dass der genaue Verlauf der Volumenentwicklung über die Zeit aufgezeichnet werden kann und nicht nur Summenvolumina zwischen weit auseinander liegenden Messungen.

Diese Maßnahmen zusammengenommen führen dazu, dass die Biogasleistung eines Biogasreaktors hochgenau erfassbar ist, auch wenn nur kleine Biogasvolumenströme vorliegen.

Für die Messung eines reproduzierbaren Biogasvolumens sind erforderlich:

a) ein konstant gehaltener Bioprozess (Überwachung, Regelung)
Der biologische Prozess und damit die Gasproduktion ist im Wesentlichen abhängig von den biologischen Parametern der eingesetzten Biomasse und der verwendeten Mikroorganismen, den physikalischen Parametern Temperatur, Durchmischung, Trockensubstanzgehalt und den chemischen Parametern pH-Wert, Redox-Potential, Beleuchtung, organische Säuren etc. Die Änderung all dieser Parameter hat Einfluss auf den biologischen Prozess und damit auf die Gasproduktion und auf das Ergebnis der Gasmessung. Die chemischen und biologischen Parameter werden daher vor dem Anlegen der Versuche konstant eingestellt. Die physikalischen Parameter müssen jedoch während der Laufzeit der Versuche geregelt und überwacht und bei der Auswertung der Versuche berücksichtigt werden. Die erfüllungsgemäße Biogas-Messeinrichtung sieht daher eine geregelte und überwachte Temperatur, einen geregelten und überwachten Rührer sowie eine Kondensation und Rückführung des Wasserdampfes aus dem Messgas zurück in die Biomasse vor.

b) Eine Messung unter konstanten Bedingungen (Standardisierung)
Hierzu wird Wasserdampf aus dem Messgas entfernt (Trocknung).

c) Eine Berücksichtigung der Umgebungsparameter (Normierung)
Bei der erfindungsgemäßen Biogas-Messeinrichtung erfolgt eine Messung und Berücksichtigung der Umgebungstemperatur und des Umgebungsdruckes.
Bei den herkömmlichen Labor-Biogasmessgeräten werden hingegen die Parameter des angeschlossenen biologischen Prozesses oder die Umgebungsbedingungen zur Auswertung der Gasmessdaten nicht berücksichtigt.

Die Berechnung des Gasvolumens erfolgt unter der Annahme eines idealen Gases durch die Erfassung des Drucks in einem konstanten Volumen. Dieses darf sich bei Änderung des Gasdruckes nicht verändern, so dass die Messkammer relativ steif ausgelegt sein muss.

Die Messsteuer- und Auswerteeinheit ist zum Messen eines aktuellen Systemdrucks nach dem Schließen des Gasauslassventils und einer Wartephase und zum Anpassen des definierten Schwellengasdrucks für einen folgenden Messzyklus im Bezug auf den aktuellen Systemdruck ausgebildet. Der definierte Schwellen-Gasdruck kann beispielsweise etwa zwei hPa über dem aktuellen Systemdruck liegen. Das heißt, wenn der Differenzdrucksensor einen Anstieg des Drucks nach Starten eines Messzyklus um den definierten relativen Schwellen-Druck erkennt, wird das Gasvolumen zu diesem Zeitpunkt ermittelt, nachdem das Gaseinlassventil geschlossen worden ist.

Die Wärmekapazität des Gehäuses, das die Messkammer umgibt, sollte sehr viel größer als die Wärmekapazität der zu messenden Gase sein. Zudem sollte die Masse des Gehäuses sehr viel größer als die Masse des von der Messkammer aufnehmbaren Gases sein. Damit wird eine möglichst große Temperaturkonstanz gewährleistet, indem die raschen Temperaturwechsel des Gases durch eine thermische Ankopplung an die Messzelle ausgeglichen werden.

Um die thermische Ankopplung an die Messzelle weiter zu optimieren, ist die Messkammer mit Stahlwolle oder ähnlichem ausgefüllt.

Vorteilhaft ist es weiterhin, wenn ein mit einem Spülgasventil gesteuerter Spülgaseinlass in der Messkammer vorgesehen ist, um ein Spülgas, wie zum Beispiel Stickstoff, in einer Spülphase in die Messkammer einzuleiten.

Die Messsteuer- und Auswerteeinheit ist vorzugsweise zum Ermitteln des Gasvolumens eines Messzyklus aus dem atmosphärischen Druck, dem Differenzdruck und dem definierten Messvolumen nach der Formel:

$$\Delta V = \left( \frac{P_a + dP}{P_a} - 1 \right) V_{REF}$$

ausgebildet.

Als Differenzdruck wird bevorzugt die Differenz des in dem Messzyklus nach Erreichen des Schwellen-Gasdrucks in der Messkammer gemessenen Gasdrucks und des in dem vorhergehenden Messzyklus ermittelten Systemdruck genutzt.

Die Normierung kann durch Berechnen eines auf einen Standarddruck, vorzugsweise $P_N$=1013,25 hPa, normierten Gasvolumens nach der Beziehung

$$\Delta V_n = \frac{P_1 \cdot \Delta V}{P_n}$$

mit dem gemessenen Druck $P_1$ in der Messkammer bei Erreichen des definierten Schwellen-Gasdrucks erfolgen.

Vorteilhaft ist es aber auch, eine auf die Standardtemperatur normiertes Gasvolumen, vorzugsweise mit einer Standardtemperatur von 20°C, nach der Beziehung

$$\Delta V_n = \frac{\Delta V \cdot T_n}{T_1}$$

mit der gemessenen Temperatur $T_1$ der Messkammer zu berechnen.

Die Messsteuer- und Auswerteeinheit ist weiterhin bevorzugt zur Integration der in aufeinander folgenden Messzyklen ermittelten Gasvolumina oder normierten Gasvolumina ausgebildet. Damit kann das in einem abgeschlossenen System erzeugte Gasvolumen über eine lange Zeit auch bei geringen Gasentwicklungen erfasst werden.

Vorzugsweise hat das Rührwerk integrierte Gasleitungen für Messgas und Spülgas, einen zylinderförmigen Rumpf mit seitlichen Bohrungen für die Gasleitungen und einer zentralen Bohrung zur Aufnahme einer Rührwelle, einen in dem Rumpf gasdicht aufgenommenen Rührmotor und einen Konus an dem Rumpf zum gasdichten Einbau in den Bioreaktor hat. Die Gasleitung für das Messgas ist dabei mit dem Gasvolumen-Messgerät verbunden. Die Gaszuleitung für das Spülgas, ist vorzugsweise durch die als Hohlwelle ausgeführte Rührwelle in den Bioreaktor geführt.

Zur Verringerung der Wasserdampfkonzentration im Messgas durch Kondensation ist vorzugsweise ein Peltier-Kühlelement in dem Rührwerk an der Gasleitung für das Messgas vorgesehen. Das Peltiert-Kühlelement kann zum Beispiel mit einer speziellen Wärmeleitfolie direkt an einen Kopf des Rührwerks gepresst werden.

Die Aufgabe wird weiterhin durch ein Verfahren mit den Merkmalen des Anspruchs 13 zur Messung von in einem Bioreaktor erzeugten Biogasvolumen mit einer solchen Biogas-Messeinrichtung mit einer Vielzahl von Messzyklen gelöst.

Vorteilhafte Ausführungsformen des erfindungsgemäßen Verfahrens sind in den Unteransprüchen beschrieben.

Die Erfindung wird nachfolgend anhand der beigefügten Zeichnungen beispielhaft näher erläutert. Es zeigen:

Figur 1 - Perspektivische Ansicht eines Gehäuses für ein in der Biogas-Messeinrichtung genutztes Gasvolumen-Messgerät;

Figur 2 - Skizze eines an einen Bioreaktor angeschlossenen Gasvolumen-Messgerätes;

Figur 3 - Flussdiagramm der Ablaufsteuerung eines Messvorgangs;

Figur 4 - Skizze eines Rührwerks für einen Bioreaktor;

Figur 5 - Schematische Darstellung der einzelnen Baugruppen der erfindungsgemäßen Biogas-Messeinrichtung zur Messung von Biogasvolumen;

Figur 6 Skizze eines Ausführungsbeispiels für einen Vergärungsmessungskolben als Bioreaktor.

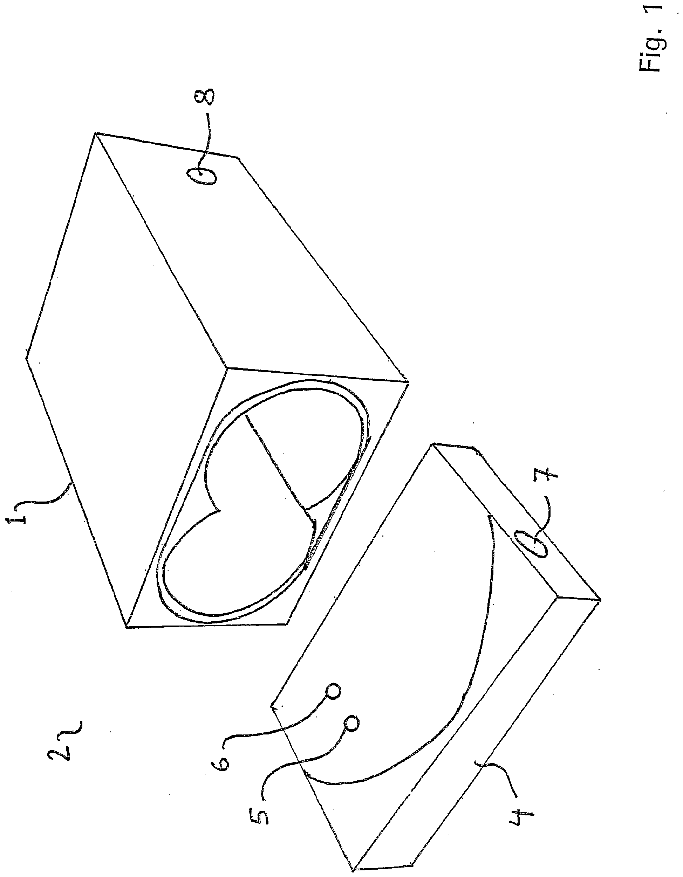

Die Figur 1 lässt eine perspektivische Ansicht eines Gehäuses 1 für ein Gasvolumen-Messgerät 2 erkennen. In dem Gehäuse 1 ist eine Messkammer 3 in Form von zwei nebeneinander liegenden und ineinander übergehenden zylindrischen Bohrungen vorgesehen. Die Messkammer 3 wird mit einer Frontplatte 4 dicht verschlossen. Hierzu kann eine O-Ringdichtung in eine Nut in dem Gehäuse 1 oder der Frontplatte 4 vorgesehen sein.

In der Frontplatte 4 ist eine Bohrung für einen Gaseinlass 5 und eine Bohrung für einen Gasauslass 6 sowie eine Durchleitbohrung 7 für Spülgase vorgesehen.

Weiterhin ist ein Differenz-Drucksensor 8 in das Gehäuse 1 eingeschraubt und kommuniziert mit der Messkammer 3 so, dass der Innendruck in der Messkammer 3 gegenüber dem Atmosphärendruck $P_a$ erfasst wird. Zur Erfassung geringster Druckdifferenzen dP ist vorzugsweise die Membran des Drucksensors 8 mit hochwertigen Elastomeren einspannungsarm gegen die Umgebung abgedichtet. Solche Elastomere können zum Beispiel Keltan, Dutral, Viton und Teflon sein. Diese Elastomere sind auch gegenüber den korrosiven Eigenschaften von Biogasen ausreichend beständig.

Das Gehäuse 1 besteht vorzugsweise aus einem massiven $V_4$A-Block, beispielsweise mit den Außenmaßen von 75 x 50 x 30 mm. Bei Ausfräsen eines definierten Volumens von 63 cm$^3$ hat das Gehäuse 1 eine Masse von 456 g. Diese Dimensionierungen sind vorteilhaft, um beim Betrieb der Messzelle die Temperaturschwankungen des Messgases zu glätten. Bis zum Erreichen eines Schwellen-Gasdrucks von beispielsweise 2 hPa oberhalb des atmosphärischen Drucks $P_a$ oder eines aktuellen Systemsdrucks $P_2$ wird das Gas geringfügig komprimiert. Da dieser Vorgang sehr langsam verläuft, kann die Kompressionswärme abgeführt werden, so dass sich das Messgas nicht erwärmt. Bei der nachfolgenden schnellen Entspannung kühlt das Gas aber ab. Wenn dann das Auslassventil geschlossen wird, ist die in der Messkammer 3 eingeschlossene Gasmasse unterkühlt. Die beim anschließenden Wärmeaustausch erfolgte Druckerhöhung resultiert jedoch aus der von außen zugeführten Wärme und nicht aus einer tatsächlichen Volumenänderung. Damit wäre das System nicht mehr geschlossen und eine Volumenberechnung nicht möglich.

Bei dem Gasvolumen-Messgerät 2 wird daher eine andere Lösung realisiert, bei der die Abkühlung des Gases im Inneren stark reduziert ist. Aufgrund der sehr viel größeren Wärmekapazität von $V_4$A im Verhältnis zur Wärmekapazität des Messgases sowie einer sehr viel größeren Masse der Messzelle im Vergleich zur Masse des Messgases kann davon ausgegangen werden, dass sich das unterkühlte Messgas bei entsprechend großer Oberfläche der Messzelle wieder erwärmt, ohne die Messzelle nennenswert abzukühlen. Durch Befüllen der Messkammer 3 mit $V_4$A-Stahlwolle und der damit verbundenen enormen Vergrößerung der inneren Oberfläche des Gasraumes steht das Messgas in einem raschen Temperaturaustausch mit dem massiven Stahlblock des Gehäuses 1, so dass anschließend keine Temperaturschwankungen mehr auftreten. Die für einen Messzyklus erforderliche Zeit lässt sich auf diese Weise auf 500 ms verkürzen.

Bei der Ausfräsung der Messkammer 3 wird ein Mittelsteg belassen, um eine möglichst hohe mechanische Stabilität des Gehäuses 1 zu gewährleisten.

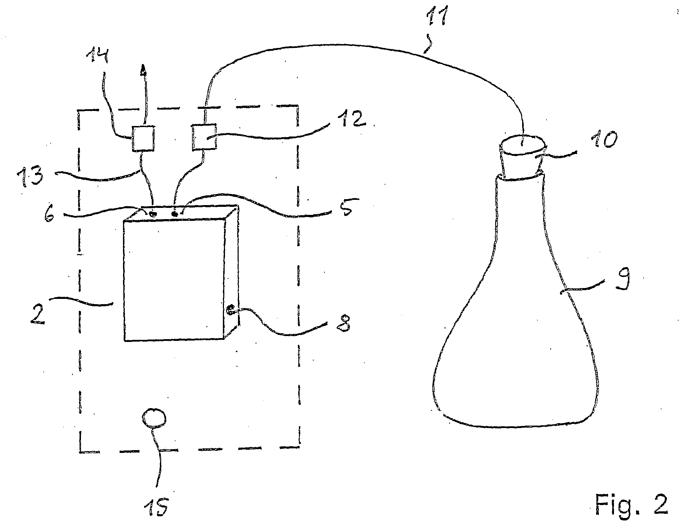

Die Figur 2 lässt den Einsatz der in der Figur 1 gezeigten Messzelle in Verbindung mit einem Bioreaktor 9 erkennen. Der Bioreaktor 9 ist beispielsweise ein Erlmeier-Kolben, in den ein Rührwerk 10 gasdicht eingeführt ist. Ein Gasableitungsschlauch 11 ist über ein Gaseinlassventil 12 zu dem Gaseinlass 5 der Messzelle geführt. Mit dem Gasauslass 6 ist eine Gasauslassleitung 13 verbunden, die über ein Gasauslassventil 14 in die Atmosphäre geführt wird.

Weiterhin ist noch ein Drucksensor 15 zur Erfassung des atmosphärischen Drucks $P_a$ vorgesehen.

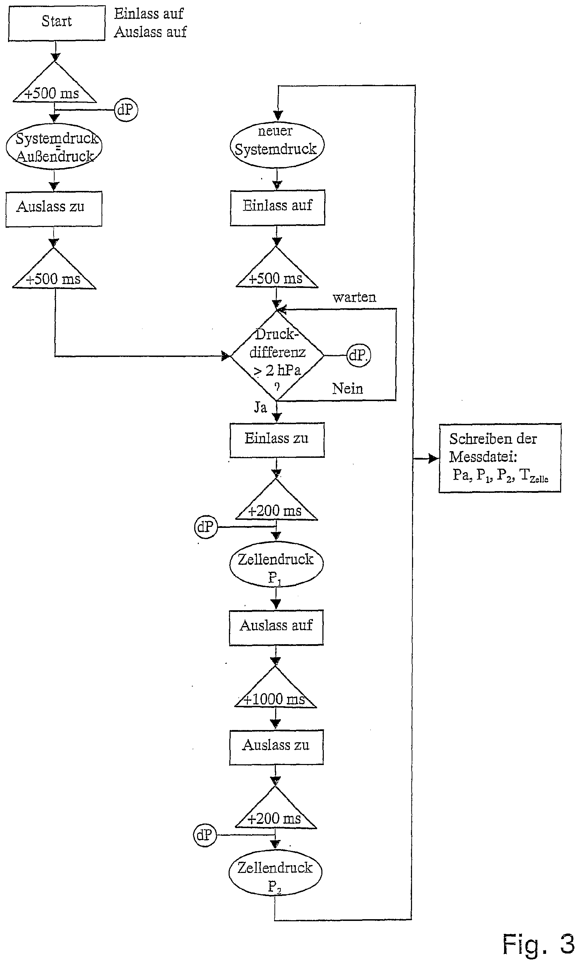

Die Durchführung einer Messung mit einem solchen System wird anhand der Figur 3 erläutert, die ein Flussdiagramm der Ablaufsteuerung des Messvorgangs zeigt. Die Messung wird wiederholt für aufeinanderfolgende Messzyklen durchgeführt.

Zunächst sind in einem Initialisierungstakt das Gaseinlassventil 12 und das Gasauslassventil 14 der Messzelle geöffnet. In dieser Stellung erfolgt 5 Sekunden nach dem Versuchsstart einmalig die Messung des relativen Innendrucks dP in der Messkammer 3 zur Feststellung eines aktuellen Systemdrucks $P_2$ und zur Kalibrierung des Systems.

Nach dem Schließen des Gasauslassventils 14 beginnt der Schalt- und Messtakt 1. Die Messkammer 3 bildet dabei mit dem Bioreaktor 9 einen geschlossenen, nach außen hin gasdichten Raum. Nach Ablauf einer Druckzeit von 500 ms fängt der Drucksensor 8 an, die Druckänderungen in der Messkammer 3, d. h. dem eingeschlossenen Gasraum zu registrieren und mit einem voreingestellten Schwellen-Gasdruck zu vergleichen. Diese Druckschwelle kann beispielsweise auf +2 hPa gesetzt werden. Mit Erreichen dieses Schwellen-Gasdrucks wird das Gaseinlassventil 12 geschlossen und ein Schalt-Messtakt 3 beginnt. Nach 200 ms erfolgt eine Messung des relativen Drucks $P_1$ in der Messkammer 3 bezogen auf den atmosphärischen Druck $P_a$. Das eingeschlossene Gasvolumen ist zu diesem Zeitpunkt durch die bekannten Parameter Druck $P_1$, Temperatur $T_1$ und Volumen V definiert. In einem Schalt- und Messtakt 3 wird das Gasauslassventil 14 für die Dauer von 1000 ms geöffnet, um das ausströmende Gas möglichst vollständig zu entspannen.

Mit dem Schließen des Gasauslassventils 14 beginnt der Schalt- und Messtakt 4, wobei nach 200 ms die zweite Messung des relativen Drucks $P_2$ in diesem Falle auf dem geringeren Druckniveau erfolgt. Dieses untere Druckniveau wird als neuer Systemdruck $P_2$ für die Berechnung des Schwellen-Gasdrucks für den nächsten Messzyklus verwendet. Der Systemdruck $P_2$ liegt in der Regel nahe am atmosphärischen Druck $P_a$ der äußeren Umgebung, kann jedoch zum Beispiel durch Gegendruck am Gasauslassventil 14 unterschiedliche Werte einnehmen, ohne das Ergebnis zu beeinflussen.

In diesem vierten Takt können alle Mittelwerte von Einzelmessungen der Temperatur $T_1$ in der Messkammer 3, des relativen Differenzdrucks $dP = P_1 - P_2$ und des atmosphärischen Drucks $P_a$ in eine Messdatei geschrieben werden.

Anschließend wird das Gaseinlassventil 12 wieder geöffnet, wodurch der Schalt- und Messtakt 1 für einen neuen Messzyklus wieder beginnt.

Die Berechnung jedes einzelnen Gasvolumens dV erfolgt nach der Gleichung

$$dV = \left( \frac{P_a + (P_1 - P_2)}{P_a} - 1 \right) V_{REF},$$

wobei $P_a$ der atmosphärische Druck, $P_1$ der im Schalt- und Messtakt 2 gemessene relative Druck $P_1$, $P_2$ der aktuelle Systemdruck und $V_{REF}$ das definierte bekannte Volumen der Messkammer 3 ist.

Dabei wird der atmosphärische Druck $P_a$ für jeden Messzyklus aktuell gemessen, so dass eine Änderung des relativen Luftdrucks nicht zu einer Fehlmessung führt.

Das in dem Bioreaktor 9 insgesamt entwickelte Biogasvolumen $V_{ges}$ setzt sich aus der Summe der in jedem Messzyklus ermittelten Einzelvolumina dV zusammen:

$$V_{ges} = \Sigma_i dV(t) \, .$$

Die Einzelgasvolumen dV oder das gesamte Gasvolumen $V_{ges}$ kann auf Standardbedingungen normiert werden. Hierdurch ist es möglich, vergleichbare Ergebnisse zu erhalten.

Das auf den Standarddruck $P_n$ normierte Gasvolumen $V_n$ berechnet sich aus dem gemessenen Druck $P_1$ und dem Standarddruck $P_n$ von 1013,25 hPa nach der Formel:

$$V_2 = \frac{P_1 \cdot V_1}{P_2} \, ,$$

wobei von einer Temperatur $T_1$ in der Messkammer 3 gleich der Standardtemperatur $T_n$ von 20°C ausgegangen wird.

Für das auf die Standardtemperatur $T_n$ von 20°C normierte Gasvolumen $V_2$ berechnet sich bei einem dem Standarddruck $P_n$ von 1013,25 hPa entsprechendem gemessenen Druck $P_1$ nach der Formel:

$$V_n = \frac{V_1 \cdot T_n}{T_1} \, .$$

Das normierte Gasvolumen $V_2$ wird in Normlitern $N_l$ angegeben.

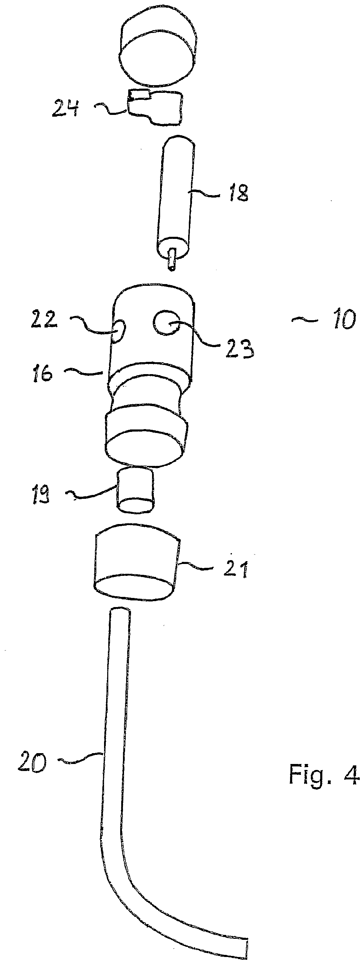

Die Figur 4 lässt ein Rührwerk 10 zum Einsatz in einem Bioreaktor 9 erkennen. Das Rührwerk 10 hat einen zylinderförmigen Rumpf 16 mit einer zentrischen Bohrung 17, in die von oben ein Rührmotor 18 und von unten eine Wellenkupplung 19 eingesetzt ist. Der Rührmotor 18 ist über die Wellenkupplung 19 mit einer gebogenen als Rohr ausgeführten Rührwelle 20 verbunden, die sich durch einen mit dem Rumpf 16 am unteren Ende verbundenen Konus 21 mit einem Stützlager erstreckt. Der Konus 21 hat an seiner Außenfläche einen Normschliff NS 29, so dass das Rührwerk 10 universell in den gängigsten Laborapparaturen aus Glas einsetzbar ist.

Der Konus 21 übernimmt die wesentliche Aufgabe bei der Abdichtung des Gasmesssystems und bei der Unterbringung der lösbaren Verbindung der Rührwelle 20 mit dem Rührmotor 18.

Die Wellenkupplung 19, die auf die Motorwelle des Rührmotors 18 verschraubt werden kann, hat einen Kugel-Verriegelungsmechanismus zur Aufnahme der Rührwelle 20. Entgegen der Drehrichtung des Rührmotors 18 verriegelt der Kugelmechanismus. Durch Drehen in Drehrichtung des Rührmotors 18 hingegen öffnet die Verriegelung und die Rührwelle 20 lässt sich entnehmen. Zur Aufnahme der Wellenkupplung 19 hat der Konus 21 eine Ausfräsung, die auch gleichzeitig zur Überleitung von Spülgas aus dem Rumpf 16 in die drehende Wellenkupplung 19 genutzt wird. Das Spülgas wird dann weiter über Bohrungen in der Wellenkupplung 19 in die hohle Rührwelle 20 geleitet. Durch das unten offene Ende der Rührwelle 19 kann das Spülgas dann bei drehendem Rührer in die Biomasse eingetragen werden. Außerdem übernimmt der Konus 21 die Weiterleitung der Messgase von dem Bioreaktor 9 in den Rumpf 16 des Motorträgers sowie die untere Lagerung und Abdichtung der Rührwelle 20.

Der Rumpf 16 hat zwei seitliche Bohrungen 22, 23 zur Führung von Messgas und Spülgas. Diese Bohrungen 22 und 23 werden im oberen Drittel des Rumpfes 16 über Gas-Schnellverbindern nach außen geführt. Aus Sicherheitsgründen sollte der Messgasausgang 22 einen im abgezogenen Zustand offenen Gasverbinder (Stecker) und der Spülgaseingang 23 einen im abgezogenen Zustand geschlossenen Gasverbinder (Buchse) haben. Auf diese Weise kann im Bioreaktor 9 entstehender Überdruck bei abgezogenen Gasleitungen jederzeit nach außen entweichen und nicht in die Spülgasbohrungen gelangen. Außerdem kann der Bioreaktor 9 so auch ohne Spülgasleitung und nur mit Messgasanschluss betrieben werden.

Die Messgasleitung ist mit einem Peltier-Kühlelement 24 als Messgaskühler verbunden, um die Wasserdampfkonzentration im Messgas durch Kondensation zu verringern. Die thermische Ankopplung der warmen Seite des Peltier-Kühlelementes 24 erfolgt vorzugsweise durch eine Wärmeleitfolie an den Kopf des Rührwerks 10.

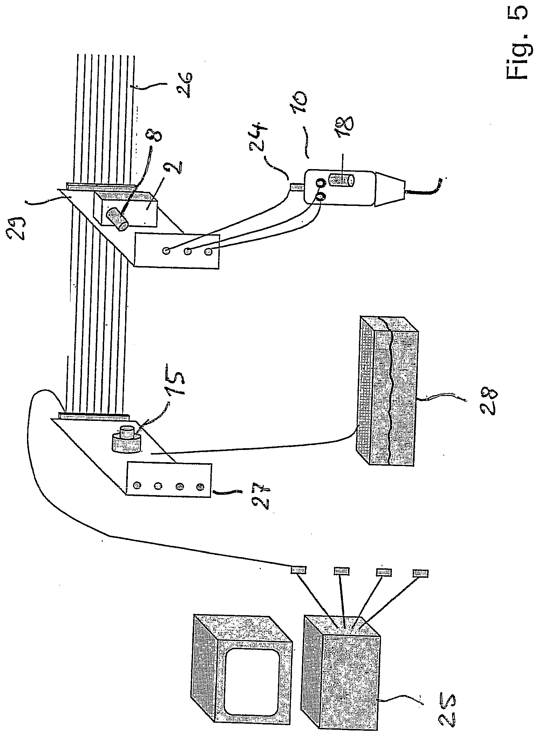

Die Figur 5 lässt eine Skizze einzelner Baugruppen eines Gasvolumen-Messsystems erkennen.

Ein Steuer- und Auswerterechner 25 ist über einen Bus 26, beispielsweise einen seriellen Bus (z. B. RS232) mit einer Steuerungskarte 27 verbunden, die den Drucksensor 15 zur Erfassung des atmosphärischen Drucks $P_a$ trägt. Die Steuerungskarte 27 dient zudem zur Überwachung der Temperatur eines Wasserbades 28, in das der Bioreaktor 9 gestellt ist.

Der Rechner 25 ist weiterhin über den Bus 26 mit einem Gasmessmodul 29 verbunden, das das Gasvolumen-Messgerät 2 mit dem Differenzdrucksensor 8 sowie die Gaseinlass- und Gasauslassventile 12, 13 trägt. Das Gasmessmodul 29 bildet weiterhin eine Schnittstelle zu den Steuerungs- und Sensorausgängen des Rührwerks 10 gemäß Figur 4.

Insbesondere wird das Peltier-Kühlelement 24 und der Rührmotor 18 angesteuert und gegebenenfalls eine Temperatur T-Peltier des Peltier-Kühlelements 24 gemessen.

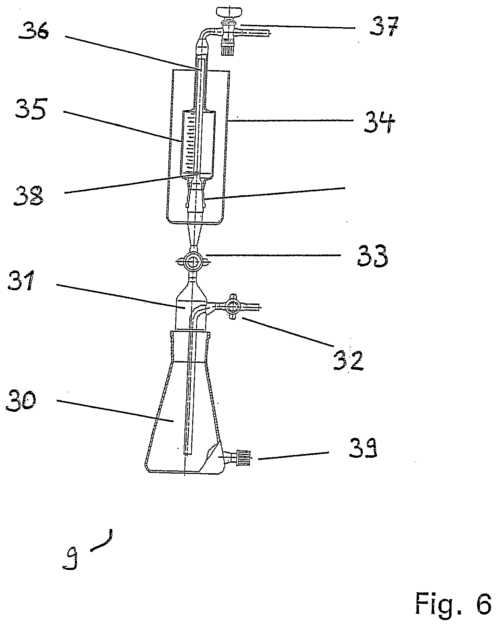

Die Figur 6 lässt einen Vergärungsmesskolben als Bioreaktor 9 zur Biogasmessung erkennen, der einen Erlenmeyerkolben 30 als Vergährungsgefäß aufweist. Der Erlenmeyerkolben 30 kann beispielsweise ein 500 ml Gefäß mit Normschliff NS45 am Anschlussrand für einen im Enmeyerkolben 30 eingesetzten Glaskonus 31 sein. Der Glaskonus 31 hat ein Stickstoff-Begasungsventil 32 für die Spülung des Biogasreaktors 9 mit Inertgas. Eine Steigleitung im Glaskonus 31 ist über ein Verbindungsventil 33 an einen in einem Wasserausgleichsgefäß 34 angeordneten skalierten Gassammelmesskolben 35 angeschlossen. Eine gasdichte Verbindung wird durch einen Normschliff NS19 an der Anschlussfläche des skalierten Gassammelkolbens 35 und des Verbindungsventilstücks 33 sichergestellt. Das im skalierten Gassammelkolben 35 angesammelte Gas wird über ein Gassteigrohr 36 und ein Entlüftungsventil 37 zum Gasvolumen-Messgerät 2 abgeführt.

Der skalierte Gassammelkolben 35 hat eine Wasseraustrittsöffnung 38.

Weiterhin ist in dem Erlenmeyerkolben 30 ein Septum 39 zur Probenentnahme vorgesehen.

Nach Inbetriebnahme des Bioreaktors 9 und Öffnung des Verbindungsventils 33 und Entlüftungsventils 37 füllt sich der Gassammelkolben 35 über die Wasseraustrittsöffnung 38 mit in dem Wasserausgleichgefäß 34 befindlichem Wasser.

Zur Einstellung anaerober Versuchsbedingungen wird Stickstoff über das Stickstoff-Begasungsventil 32 im Erlenmeyerkolben 30 befindliche Biomasse geleitet. Der Stickstoff kann durch das Gassteigrohr 36 und das Entlüftungsventil 37 aus dem Biogasreaktor 9 entweichen. Anschließend werden das Begasungs- und Entlüftungsventil 32, 37 geschlossen und die Gasmessung gestartet. Dabei wird das Entlüftungsventil und das Gasvolumen-Messgerät angeschlossen und zur kontinuierlichen Gasmessung wieder geöffnet.

**Patentansprüche**

1.   Biogas-Messeinrichtung mit:

    - einem Bioreaktor (9),
    - einem kommunizierend mit dem Bioreaktor (9) über eine Gasleitung (11) für Biogas verbundenen Gasvolumen-Messgerät (2) mit:

        - einer Messkammer (3), die ein definiertes Messvolumen ($V_{REF}$) in einem Gehäuse (1) hat,
        - einem mit einem Gaseinlassventil (12) gesteuerten Gaseinlass (5) in die Messkammer (3),
- einem mit einem Gasauslassventil (14) gesteuerten Gasauslass (6) aus der Messkammer (3),
- einem Drucksensor (8) zur Erfassung des atmosphärischen Drucks ($P_a$),
- einem mit der Messkammer (3) kommunizierenden Differenzdrucksensor (8) zur Erfassung des Differenzdrucks (dP) zwischen dem Gasdruck in der Messkammer (3) und dem atmosphärischen Druck ($P_a$) oder einem aktuellen Systemdruck (P2),
- einer Mess-Steuer- und Auswerteeinheit, die zur Steuerung der Biogas-Messeinrichtung und Ermittlung der im Bioreaktor erzeugten Gasvolumen (V) in Abhängigkeit von mit dem Gasvolumen-Messgerät (2) gemessenen Gasvolumen ($\Delta V$) aufeinander folgender Messzyklen mit den Schritten für jeden Messzyklus von:

    f) Einleiten einer Gasmenge in die Messkammer (3) durch Öffnen des Gaseinlassventils (12) solange, bis ein definierter Schwellen-Gasdruck erreicht ist,
    g) Schließen des Gaseinlassventils (12), wenn ein definierter Schwellen-Gasdruck erreicht ist,
    h) Messen der Temperatur ($T_1$) in der Messkammer (3),
    i) Öffnen des Gasauslassventils (14) für eine Entspannungszeit,
    j) Schließen des Gasauslassventils (14), und
    k) Messen des aktuellen Systemdrucks ($P_2$) in der Messkammer (3) nach einer Wartephase eingerichtet ist, wobei

- der Bioreaktor (9) nach außen gasdicht so abgeschlossen ist, dass sämtliche in dem Bioreaktor (9) erzeugten Gasvolumina durch die Messkammer (3) des Gasvolumen-Messgerätes (2) erfassbar sind,
- ein Rührwerk (10) gasdicht in den Bioreaktor geführt ist,
- ein Kühlelement an der Gasleitung (11) für das Biogas vorgesehen ist, und
- die Mess-Steuer- und Auswerteeinheit zur Steuerung der Biogas-Messeinrichtung derart ausgebildet ist, dass der Schritt h) nach einer Wartephase zusammen mit einer Messung des relativen Drucks ($P_1$) in der Messkammer (3) bezogen auf den atmosphärischen Druck ($P_a$) erfolgt, dass das Kühlelement zur Verringerung der Wasserdampfkonzentration im Biogas durch Kondensation angesteuert wird und dass das

gemessene Gasvolumen ($\Delta V$) eines Messzyklus auf Standardbedingungen normiert wird,
**dadurch gekennzeichnet, dass**
- die Messkammer (3) des Gasvolumen-Messgerätes (2) mit Stahlwolle ausgefüllt ist.

2. Biogas-Messeinrichtung nach Anspruch 1, **dadurch gekennzeichnet, dass** der definierte Schwellen-Gasdruck etwa 2 hPa über einem aktuellen Systemdruck ($P_2$) beträgt.

3. Biogas-Messeinrichtung nach einem der vorhergehenden Ansprüche, **dadurch gekennzeichnet, dass** die Wärmekapazität des Gehäuses (1) des Gasvolumen-Messgerätes (2) sehr viel größer als die Wärmekapazität der zu messenden Gase und die Masse des Gehäuses (1) sehr viel größer als die Masse des von der Messkammer (3) aufnehmbaren Gases ist.

4. Biogas-Messeinrichtung nach einem der vorhergehenden Ansprüche, **gekennzeichnet durch** einen mit einem Spülgasventil gesteuerten Spülgaseinlass in der Messkammer (3) des Gasvolumen-Messgerätes (2) zum Einleiten eines Spülgases, insbesondere von Stickstoff ($N_2$).

5. Biogas-Messeinrichtung nach einem der vorhergehenden Ansprüche, **dadurch gekennzeichnet, dass** die Messsteuer- und Auswerteeinheit zum Ermitteln des Gasvolumens ($\Delta V$) eines Messzyklus aus dem atmosphärischen Druck ($P_a$) dem Differenzdruck ($dP$) und dem definierten Messvolumen ($V_{REF}$) nach der Gleichung

$$\Delta V = \left( \frac{P_a + dP}{P_a} - 1 \right) V_{REF}$$

ausgebildet ist.

6. Biogas-Messeinrichtung nach einem der vorhergehenden Ansprüche, **dadurch gekennzeichnet, dass** die Messsteuer- und Auswerteeinheit zum Berechnen eines auf einen Standarddruck ($P_n$), vorzugsweise mit $P_n$ =1.013,25 hPa, normierten Gasvolumens ($\Delta V_n$) nach der Beziehung

$$\Delta V_n = \frac{P_1 \cdot \Delta V}{P_n},$$

wobei $P_1$ der nach dem Schritt g) und einer Wartephase gemessene relative Druck in der Messkammer (3) ist, ausgebildet ist.

7. Biogas-Messeinrichtung nach einem der vorhergehenden Ansprüche, **gekennzeichnet durch** einen Temperatursensor, der zur Messung der Temperatur ($T_1$) der Messkammer (3) in Verbindung mit dem Gehäuse (1) des Gasvolumen-Messgerätes (2) steht.

8. Biogas-Messeinrichtung nach Anspruch 9, **dadurch gekennzeichnet, dass** die Messsteuer- und Auswerteeinheit zum Berechnen eines auf eine Standardtemperatur ($T_n$), vorzugsweise mit $T_n$=20°C, normierten Gasvolumens ($\Delta V_n$) nach der Beziehung

$$\Delta V_n = \frac{\Delta V \cdot T_n}{T_1}$$

mit der gemessenen Temperatur ($T_1$) der Messkammer (3) ausgebildet ist.

9. Biogas-Messeinrichtung nach einem der vorhergehenden Ansprüche, **dadurch gekennzeichnet, dass** die Messsteuer- und Auswerteeinheit zur Integration der in aufeinander folgenden Messzyklen ermittelten Gasvolumina ($\Delta V$) ausgebildet ist.

10. Biogas-Messeinrichtung nach Anspruch 9, **dadurch gekennzeichnet, dass** das Rührwerk (10) integrierte Gasleitungen (11 , 13) für Biogas und Spülgas, einen zylinderförmigen Rumpf (16) mit seitlichen Bohrungen (22, 23) für die Gasleitungen (11, 13) und mit einer zentralen Bohrung zur Aufnahme einer Rührwelle (20), einen in dem Rumpf (16) gasdicht aufgenommenen Rührmotor (18) und einen Konus (21) an dem Rumpf (16) zum gasdichten Einbau in den Bioreaktor (9) hat, wobei die Gasleitung (11, 13) für das Biogas mit dem Gasvolumen-Messgerät (2) verbunden ist.

11. Gasvolumen-Messgerät (2) nach Anspruch 10, **dadurch gekennzeichnet, dass** eine Gaszuleitung (13) durch die als Hohlwelle ausgeführte Rührwelle (20) in dem Bioreaktor (9) geführt ist.

12. Gasvolumen-Messgerät (2) nach einem der Ansprüche 10 und 11, **dadurch gekennzeichnet, dass** das Kühlelement ein Peltier-Kühlelement (24) ist.

13. Verfahren zur Messung von in einem Bioreaktor (9) erzeugten Biogasvolumen mit einer Biogas-Messeinrichtung nach einem der vorhergehenden Ansprüche mit einer Vielzahl von Messzyklen, für jeden Messzyklus **gekennzeichnet durch** die Schritte:

    h) Einleiten einer Gasmenge von Biogas in die Messkammer (3) durch Öffnen des Gaseinlassventils (12) solange, bis ein definierter Schwellen-Gasdruck erreicht ist,

i) Schließen des Gaseinlassventils (12), wenn ein definierter Schwellen-Gasdruck erreicht ist,

j) Messen der Temperatur ($T_1$) in der Messkammer (3),

k) Öffnen des Gasauslassventils (14) für eine Entspannungszeit, und

l) Schließen des Gasauslassventils (14),

m) Messen des aktuellen Systemdrucks ($P_2$) in der Messkammer (3) nach einer Wartephase,

**gekennzeichnet durch**

- Kühlen des Biogases in der Gasleitung (11) für das Biogas mit dem Kühlelement zur Verringerung der Wasserdampfkonzentration im Biogas durch Kondensation,

- Durchführung des Schritts h) nach einer Wartephase zusammen mit einer Messung des relativen Drucks ($P_1$) in der Messkammer (3) bezogen auf den atmosphärischen Druck ($P_a$),

- Ermitteln des Gasvolumens ($\Delta V$) eines Messzyklus aus dem atmosphärischen Druck ($P_a$) dem Differenzdruck (dP) und dem definierten Messvolumen ($V_{REF}$) nach der Gleichung

$$\Delta V = \left( \frac{P_a + dP}{P_a} - 1 \right) V_{REF}$$

und

- Berechnen eines auf einen Standarddruck ($P_n$), vorzugsweise mit $P_n$ = 1.013,25 hPa, normierten Gasvolumens ($\Delta V_n$) nach der Beziehung

$$\Delta V_n = \frac{P_1 \cdot \Delta V}{P_n},$$

wobei $P_1$ der nach dem Schritt i) und einer Wartephase gemessene relative Druck in der Messkammer (3) ist, zur Normierung des gemessenen Gasvolumens ($\Delta V$) eines Messzyklus auf Standardbedingungen.

14. Verfahren nach Anspruch 13, **gekennzeichnet durch** Berechnen eines auf eine Standardtemperatur ($T_n$), vorzugsweise mit $T_n$=20°C, normierten Gasvolumens ($\Delta V_n$) nach der Beziehung

$$\Delta V_n = \frac{\Delta V \cdot T_n}{T_1}$$

mit der gemessenen Temperatur ($T_1$) der Messkammer (3).

**Claims**

1. Biogas measuring device with:

- a bioreactor (9),
- a gas volume meter (2) connected communicatingly to the bioreactor (9) via a gas conduit (11) for biogas, with:

  - a measuring chamber (3) which has a defined measurement volume ($V_{REF}$) in a housing (1),
  - a gas inlet (5) into the measuring chamber (3), said gas inlet being controlled by a gas inlet valve (12),
  - a gas outlet (6) from the measuring chamber (3), said gas outlet being controlled by a gas outlet valve (14),
  - a pressure sensor (8) for detecting the atmospheric pressure ($P_a$),
  - a differential pressure sensor (8), communicating with the measuring chamber (3), for detecting the differential pressure (dP) between the gas pressure in the measuring chamber (3) and the atmospheric pressure ($P_a$) or a current system pressure ($P_2$),
  - a measurement, control and evaluation unit which is set up for controlling the biogas measuring device and determining the gas volumes (V) generated in the bioreactor as a function of gas volumes ($\Delta V$), measured by the gas volume meter (2), of successive measurement cycles, with the steps for each measurement cycle of:

    f) introduction of a gas quantity into the measuring chamber (3) by opening the gas inlet valve (12) until a defined threshold gas pressure is reached,

    g) closing of the gas inlet valve (12) when a defined threshold gas pressure is reached,

    h) measurement of the temperature ($T_1$) in the measuring chamber (3),

    i) opening of the gas outlet valve (14) for an expansion time,

    j) closing of the gas outlet valve (14), and

    k) measurement of the current system pressure ($P_2$) in the measuring chamber (3) after a standby phase,

  **wherein**

  - the bioreactor (9) is closed off, outwardly gastight, such that all the gas volumes generated in the bioreactor (9) can be detected by the measuring chamber (3) of the gas volume meter (2),

- an agitator (10) is guided, gastight, into the bioreactor,
- a cooling element is provided on the gas conduit (11) for the biogas, and
- the measurement, control and evaluation unit for controlling the biogas measuring device is designed in such a way that step h) is performed after a standby phase together with a measurement of the relative pressure ($P_1$) in the measuring chamber (3) relative to atmospheric pressure ($P_a$), that the cooling element is activated in order to reduce the water vapor concentration in the biogas due to condensation and that the measured gas volume ($\Delta V$) of a measurement cycle is normalized to standard conditions, is

**characterized in that**

- the measuring chamber (3) of the gas volume meter (2) is filled with steel wool.

2. Biogas measuring device according to Claim 1, **characterized in that** the defined threshold gas pressure amounts to about 2 hPa above a current system pressure ($P_2$).

3. Biogas measuring device according to either of the preceding claims, **characterized in that** the heat capacity of the housing (1) of the gas volume meter (2) is very much higher than the heat capacity of the gases to be measured and the mass of the housing (1) is very much greater than the mass of the gas capable of being received by the measuring chamber (3).

4. Biogas measuring device according to one of the preceding claims, **characterized by** a scavenging gas inlet, controlled by a scavenging gas valve, in the measuring chamber (3) of the gas volume meter (2), for the introduction of a scavenging gas, in particular of nitrogen ($N_2$).

5. Biogas measuring device according to one of the preceding claims, **characterized in that** the measurement, control and evaluation unit is designed for determining the gas volume ($\Delta V$) of a measurement cycle from the atmospheric pressure ($P_a$), the differential pressure (dP) and the defined measurement volume ($V_{REF}$) according to the equation

$$\Delta V = \left( \frac{P_a + dP}{P_a} - 1 \right) V_{REF} .$$

6. Biogas measuring device according to one of the preceding claims, **characterized in that** the measurement, control and evaluation unit is designed for calculating a gas volume ($\Delta V_n$) normalized to a standard pressure ($P_n$), preferably with $P_n$ = 1013.25 hPa, according to the relation

$$\Delta V_n = \frac{P_1 \cdot \Delta V}{P_n} ,$$

wherein $P_1$ is the relative pressure in the measuring chamber (3) measured after step g) and a standby phase,

7. Biogas measuring device according to one of the preceding claims, **characterized by** a temperature sensor which to measure the temperature ($T_1$) of the measuring chamber (3) is connected to the housing (1) of the gas volume meter (2).

8. Biogas measuring device according to Claim 9, **characterized in that** the measurement, control and evaluation unit is designed for calculating a gas volume ($\Delta V_n$) normalized to a standard temperature ($T_n$), preferably with $T_n$ = 20°C, according to the relation

$$\Delta V_n = \frac{\Delta V \cdot T_n}{T_1}$$

by means of the measured temperature ($T_1$) of the measuring chamber (3).

9. Biogas measuring device according to one of the preceding claims, **characterized in that** the measurement, control and evaluation unit is designed for integrating the gas volumes ($\Delta V$) determined in successive measurement cycles.

10. Biogas measuring device according to Claim 9, **characterized in that** the agitator (10) has integrated gas conduits (11, 13) for biogas and scavenging gas, a cylindrical body (16) with lateral bores (22, 23) for the gas conduits (11, 13) and with a central bore for receiving an agitating shaft (20), an agitating motor (18) received, gastight, in the body (16), and a cone (21) on the body (16) for gastight installation in the bioreactor (9), the gas conduit (11, 13) for the biogas being connected to the gas volume meter (2).

11. Gas volume meter (2) according to Claim 10, **characterized in that** a gas supply conduit (13) is led in the bioreactor (9) through the agitating shaft (20) designed as a hollow shaft.

12. Gas volume meter (2) according to one of Claims 10 and 11, **characterized in that** the cooling element is a Peltier cooling element (24).

**13.** Method for measuring biogas volumes generated in a bioreactor (9), by means of a biogas measuring device according to one of the preceding claims, with a multiplicity of measurement cycles, characterized for each measurement cycle by the steps of:

h) introduction of a gas quantity of biogas into the measuring chamber (3) by opening the gas inlet valve (12) until a defined threshold gas pressure is reached,
i) closing of the gas inlet valve (12) when a defined threshold gas pressure is reached,
j) measurement of the temperature ($T_1$) in the measuring chamber (3),
k) opening of the gas outlet valve (14) for an expansion time, and
1) closing of the gas outlet valve (14),
m) measurement of the current system pressure ($P_2$) in the measuring chamber (3) after a standby phase,

**characterized by**

- cooling of the biogas in the gas conduit (11) for the biogas by means of the cooling element in order to reduce the water vapor concentration in the biogas due to condensation,
- performing step h) after a standby phase together with a measurement of the relative pressure ($P_1$) in the measuring chamber (3) relative to atmospheric pressure ($P_a$),
- determining the gas volume ($\Delta V$) of a measurement cycle from the atmospheric pressure ($P_a$), the differential pressure (dP) and the defined measurement volume ($V_{REF}$) according to the equation

$$\Delta V = \left( \frac{P_a + dP}{P_a} - 1 \right) V_{REF}$$

and

- calculation of a gas volume ($\Delta V_n$) normalized to a standard pressure ($P_n$), preferably with $P_n$ = 1013.25 hPa, according to the relation

$$\Delta V_n = \frac{P_1 \cdot \Delta V}{P_n},$$

wherein $P_1$ is the relative pressure in the measuring chamber (3) measured after step i) and a standby phase, for normalization of the measured gas volume ($\Delta V$) of a measurement cycle to standard conditions.

**14.** Method according to Claim 13, **characterized by** the calculation of a gas volume ($\Delta V_n$) normalized to a standard temperature ($T_n$), preferably with $T_n$ = 20°C, according to the relation

$$\Delta V_n = \frac{\Delta V \cdot T_n}{T_1}$$

by means of the measured temperature ($T_1$) of the measuring chamber (3).

**Revendications**

**1.** Dispositif de mesure de biogaz présentant :

un bioréacteur (9) et
un appareil (2) de mesure de volume de gaz relié de manière à communiquer avec le bioréacteur (9) par l'intermédiaire d'un conduit (11) à biogaz, l'appareil présentant :

une chambre de mesure (3) qui possède un volume de mesure ($V_{REF}$) défini dans un boîtier (1),
une entrée de gaz (5) dans la chambre de mesure (3), commandée par une soupape (12) d'entrée de gaz,
une sortie de gaz (6) hors de la chambre de mesure (3), commandée par une soupape (14) de sortie de gaz,
un capteur de pression (8) qui saisit la pression atmosphérique ($P_a$),
un capteur (8) de pression différentielle communiquant avec la chambre de mesure (3) en vue de saisir la différence de pression (dP) entre la pression de gaz qui règne dans la chambre de mesure (3) et la pression atmosphérique ($P_a$) ou une pression effective (P2) du système,
une unité de commande de mesure et d'évaluation conçue pour commander le dispositif de mesure de biogaz et pour déterminer les volumes (V) de gaz produits dans le bioréacteur en fonction de cycles de mesure successifs de volumes de gaz ($\Delta V$) mesurés à l'aide de l'appareil (2) de mesure de volume de gaz, chaque cycle de mesure présentant les étapes suivantes :

f) introduire une quantité de gaz dans la chambre de mesure (3) par ouverture de la soupape (12) d'entrée de gaz jusqu'à ce qu'un seuil défini de pression de gaz soit atteint,
g) fermer la soupape (12) d'entrée de gaz lorsqu'un seuil défini de pression de gaz a été atteint,

h) mesurer la température ($T_1$) qui règne dans la chambre de mesure (3),

i) ouvrir la soupape (14) de sortie de gaz pendant une durée de détente,

j) fermer la soupape (14) de sortie de gaz et

k) mesurer la pression effective ($P_2$) du système qui règne dans la chambre de mesure (3) après une phase d'attente,

le bioréacteur (9) étant fermé vis-à-vis de l'extérieur de manière étanche aux gaz de telle sorte que tous les volumes de gaz formés dans le bioréacteur (9) puissent être saisis par la chambre de mesure (3) de l'appareil (2) de mesure de volume de gaz, un mécanisme de brassage (10) étant guidé de manière étanche dans le bioréacteur, un élément de refroidissement étant prévu sur le conduit (11) à biogaz, l'unité de commande de mesure et d'évaluation qui commande le dispositif de mesure de biogaz étant configurée de telle sorte que l'étape h) est exécutée après une phase d'attente en même temps qu'une mesure de la pression relative ($P_1$) qui règne dans la chambre de mesure (3) par rapport à la pression atmosphérique ($P_a$), de telle sorte que l'élément de refroidissement soit commandé pour diminuer la condensation la concentration en vapeur d'eau dans le biogaz et que le volume de gaz ($\Delta V$) mesuré soit normé par rapport à des conditions standard au cours d'un cycle de mesure, est **caractérisé en ce que** la chambre de mesure (3) de l'appareil (2) de mesure de volume de gaz est remplie de laine d'acier.

**2.** Dispositif de mesure de biogaz selon la revendication 1, **caractérisé en ce que** le seuil défini de pression de gaz est situé à environ 2 hPa au-dessus de la pression effective du système ($P_2$).

**3.** Dispositif de mesure de biogaz selon l'une des revendications précédentes, **caractérisé en ce que** la capacité calorifique du boîtier (1) de l'appareil (2) de mesure de volume de gaz est plusieurs fois plus élevée que la capacité calorifique des gaz à mesurer et **en ce que** la masse du boîtier (1) est plusieurs fois plus grande que la masse du gaz que peut reprendre la chambre de mesure (3).

**4.** Dispositif de mesure de biogaz selon l'une des revendications précédentes, **caractérisé par** une introduction de gaz de balayage, commandée par une soupape à gaz de balayage, dans la chambre de mesure (3) de l'appareil (2) de mesure de volume de gaz, pour introduire un gaz de balayage en particulier de l'azote ($N_2$).

**5.** Dispositif de mesure de biogaz selon l'une des revendications précédentes, **caractérisé en ce que** l'unité de commande de mesure et d'évaluation qui détermine le volume de gaz ($\Delta V$) d'un cycle de mesure à partir de la pression atmosphérique ($P_a$), de la pression différentielle (dP) et du volume de mesure ($V_{REF}$) défini est formée selon l'équation

$$\Delta V = \left( \frac{P_a + dP}{P_a} - 1 \right) V_{REF}$$

.

**6.** Dispositif de mesure de biogaz selon l'une des revendications précédentes, **caractérisé en ce que** l'unité de commande de mesure et d'évaluation est configurée pour calculer un volume de gaz ($\Delta V_n$) normé par rapport à une pression standard ($P_n$), de préférence avec $P_n = 1\,013,25$ hPa, selon l'équation

$$\Delta V_n = \frac{P_1 \cdot \Delta V}{P_n}$$

dans laquelle $P_1$ est la pression relative qui règne dans la chambre de mesure (3) et mesurée après l'étape g) et une phase d'attente.

**7.** Dispositif de mesure de biogaz selon l'une des revendications précédentes, **caractérisé par** un capteur de température qui sert à mesurer la température ($T_1$) de la chambre de mesure (3) en association avec le boîtier (1) de l'appareil (2) de mesure de volume de gaz.

**8.** Dispositif de mesure de biogaz selon la revendication 9, **caractérisé en ce que** l'unité de commande et mesure et d'évaluation est configurée pour calculer un volume de gaz ($\Delta V_n$) normé par rapport à une température standard ($T_n$), de préférence avec $T_n = 20°C$, selon l'équation

$$\Delta V_n = \frac{\Delta V \cdot T_n}{T_1}$$

dans laquelle ($T_1$) est la température mesurée dans la chambre de mesure (3).

**9.** Dispositif de mesure de biogaz selon l'une des revendications précédentes, **caractérisé en ce que** l'unité de commande de mesure et d'évaluation est formée pour intégrer les volumes de gaz (ΔV) déterminés dans des cycles de mesure successifs.

**10.** Dispositif de mesure de biogaz selon la revendication 9, **caractérisé en ce que** le mécanisme de brassage (10) possède des conduits de gaz (11, 13) intégrés pour le biogaz et le gaz de balayage, un conducteur cylindrique (16) doté d'alésages latéraux (22, 23) pour les conduits de gaz (11, 13) et d'un alésage central qui reprend un arbre de brassage (20), un moteur (18) de brassage reçu de manière étanche aux gaz dans le collecteur (16) et un cône (21) prévu sur le collecteur (16) pour être monté de manière étanche aux gaz dans le bioréacteur (9), le conduit de gaz (11, 13) prévu pour le biogaz étant relié à l'appareil (2) de mesure de volume de gaz.

**11.** Appareil (2) de mesure de volumes de gaz selon la revendication 10, **caractérisé en ce qu'**un conduit (13) d'amenée de gaz est guidé comme arbre de brassage (20) réalisé comme arbre creux dans le bioréacteur (9).

**12.** Appareil (2) de mesure de volumes de gaz selon l'une des revendications 10 et 11, **caractérisé en ce que** l'élément de refroidissement est un élément de refroidissement Peltier (24).

**13.** Procédé de mesure de volumes de biogaz formés dans un bioréacteur (9) à l'aide d'un dispositif de mesure de biogaz selon l'une des revendications précédentes, présentant plusieurs cycles de mesure, chaque cycle de mesure étant **caractérisé par** les étapes qui consistent à :

h) introduire une quantité de gaz dans la chambre de mesure (3) par ouverture de la soupape (12) d'entrée de gaz jusqu'à ce qu'un seuil défini de pression de gaz soit atteint,
i) fermer la soupape (12) d'entrée de gaz lorsqu'un seuil défini de pression de gaz a été atteint,
j) mesurer la température ($T_1$) qui règne dans la chambre de mesure (3),
k) ouvrir la soupape (14) de sortie de gaz pendant une durée de détente,
l) fermer la soupape (14) de sortie de gaz et
m) mesurer la pression effective ($P_2$) du système qui règne dans la chambre de mesure (3) après une phase d'attente,

**caractérisé par** :

le refroidissement du biogaz dans le conduit à biogaz (11) à l'aide de l'élément de refroidissement en vue de diminuer par condensation la concentration du biogaz en vapeur d'eau,
l'exécution de l'étape h) après une phase d'attente, en même temps que la mesure de la pression relative ($P_1$) qui règne dans la chambre de mesure (3) par rapport à la pression atmosphérique ($P_a$),
la détermination du volume de gaz (ΔV) d'un cycle de mesure à partir de la pression atmosphérique ($P_a$), de la pression différentielle (dP) et du volume de mesure ($V_{REF}$) défini selon l'équation

$$\Delta V = \left( \frac{P_a + dP}{P_a} - 1 \right) V_{REF}$$

et
le calcul d'un volume de gaz ($\Delta V_n$) normé par rapport à une pression standard ($P_n$), de préférence avec $P_n = 1\,013{,}25$ hPa, selon l'équation

$$\Delta V_n = \frac{P_1 \cdot \Delta V}{P_n}$$

dans laquelle $P_1$ est la pression qui règne dans la chambre de mesure (3) et mesurée après l'étape i) et une phase d'attente, en vue de normer par rapport à des conditions standard le volume de gaz (ΔV) d'un cycle de mesure.

**14.** Procédé selon la revendication 13, **caractérisé par** le calcul d'un volume de gaz ($\Delta V_n$) normé par rapport à une température standard ($T_n$), de préférence avec $T_n = 20°C$, selon l'équation

$$\Delta V_n = \frac{\Delta V \cdot T_n}{T_1}$$

dans laquelle ($T_1$) est la température mesurée dans la chambre de mesure (3).

Fig. 1

Fig. 2

Fig. 3

24

18

22

16

10

23

19

21

20

Fig. 4

Fig. 5

EP 1 794 550 B1

36
37
35
34
38
33
31
32
30
39

9

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5742523 A
- FR 2767206
- DE 10162286 A1
- EP 0021470 A1
- EP 497414 A

**In der Beschreibung aufgeführte Nicht-Patentliteratur**

- **BÜCHS J. ; ANDERLEI, T.** RAMOS (Respiration Activity Monitoring System) - Online - Messung der Atmungsaktivitäten biologischer Kulturen in geschüttelten Bioreaktoren. *BIOforum,* Marz 2001, 149-151